# EUROPEAN PATENT APPLICATION

(11) **EP 4 273 873 A1**
(43) Date of publication of application: **08.11.2023**
(21) Application number: 22211960.4
(22) Date of filing: 07.12.2022
(51) Int. Cl.: G16H 20/30

(54) **PERSONALIZED EXERCISE GUIDANCE SYSTEM AND METHOD BASED ON MACHINE LEARNING**

(30) Priority: 05.05.2022 CN 202210477547
(71) Applicant: Chengdu Shangyi Information Technology Co., Ltd., Chengdu, Sichuan 610000 (CN)
(72) Inventor: LEI, Zhen, Chengdu, 610000 (CN); HE, Chunshui, Chengdu, 610000 (CN); CHEN, Xi, Chengdu, 610000 (CN); ZENG, Ling, Chengdu, 610000 (CN); DONG, Lei, Chengdu, 610000 (CN); FAN, Yi, Chengdu, 610000 (CN)
(74) Representative: M. Zardi & Co S.A.

(57) **Abstract**

The present disclosure relates to a personalized exercise guidance system and method based on machine learning. The personalized exercise guidance system includes: a server, a doctor client, and a patient client. The patient client is configured to obtain basic data, evaluation data, and exercise behavior data of a patient, and send the basic data, the evaluation data, and the exercise behavior data to the server. The doctor client is configured to determine an exercise prescription according to the basic data and medical record data of the patient, and upload the exercise prescription and the medical record data to the server. The server is configured to determine an exercise effect according the evaluation data and the exercise behavior data of the patient.

## Description

### TECHNICAL FIELD

The present disclosure relates to the technical field of machine learning, and in particular, to a personalized exercise guidance system and method based on machine learning.

### BACKGROUND ART

With the development of the times, mass fitness has been greatly developed in recent years, and the masses are aware of the importance of exercise for physical health. The mass fitness has spawned a variety of exercise software, whose main functions are mainly limited to providing exercise prescriptions based on basic information, such as gender, age, height, and weight, and training goals of the patient according to the template content preset in the backend.

Exercise guidance is essential for public health, chronic disease management, and disease rehabilitation. Universal exercise guidance is not suitable for people with diseases. With the improvement of living standards and health awareness, personalized exercise guidance is needed even for public health.

Scientific personalized exercise prescriptions require relevant professionals to perform comprehensive evaluation of basic health indicators, living habits, disease states, exercise function, and physical fitness, and then formulate personalized exercise rehabilitation courses and perform one-on-one guidance based on evaluation results. This requires professionals to have a lot of professional knowledge and corresponding clinical experience. Therefore, the establishment of scientific personalized exercise prescriptions has a high threshold and a large workload, which brings high costs and cannot meet the needs of exercise therapy and rehabilitation for a large number of people with diseases.

### SUMMARY

An objective of the present disclosure is to provide a personalized exercise guidance system and method based on machine learning, which can improve the accuracy and pertinence of exercise guidance.

In order to achieve the above objective, the present disclosure provides the following technical solutions:

A personalized exercise guidance system based on machine learning includes: a server, a doctor client, and a patient client.

The patient client is configured to obtain basic data, evaluation data, and exercise behavior data of a patient, and send the basic data, the evaluation data, and the exercise behavior data to the server. The basic data includes: gender, age, weight, living habits, exercise habits, diseases, pain conditions, and exercise risk information. The evaluation data includes: resting heart rate, cardiopulmonary endurance, upper limb and lower limb muscle strength, core muscle strength, upper limb and lower limb flexibility, balance, coordination, and musculoskeletal ability before and after an exercise prescription. The exercise behavior data includes: exercise time, exercise actions, heart rate during exercise, and physiological indicators before, during, and after exercise.

The doctor client is configured to determine the exercise prescription according to the basic data and medical record data of the patient, and upload the exercise prescription and the medical record data to the server. The medical record data includes: disease symptoms, physical signs, key indicators, and examination reports at different time points.

The server is configured to determine an exercise effect according the evaluation data and the exercise behavior data of the patient, generate a patient eigenvector from the basic data, initial evaluation data, and the medical record data of the patient, and a prescription eigenvector from the corresponding exercise prescription, and construct a prescription generation model using a machine learning method according to the patient eigenvector, the prescription eigenvector, and the exercise effect. The prescription generation model is configured to take the patient eigenvector as an input and the exercise prescription as an output.

Optionally, the server may include: a data pre-processing module.

The data pre-processing module may be configured to perform data pre-processing on the basic data, the evaluation data, the exercise behavior data, the medical record data, and the exercise prescription. The data pre-processing may include: data cleaning and data normalization.

Optionally, the server may include: a storage module.

The storage module may be configured to store the basic data, the evaluation data, the exercise behavior data, the medical record data, the exercise prescription, and the exercise effect of the patient.

A personalized exercise guidance method based on machine learning, applied to the personalized exercise guidance system based on machine learning, includes:
obtaining the basic data, the evaluation data, and the exercise behavior data of the patient, and generating the exercise prescription according to the basic data and the medical record data of the patient;
determining the exercise effect according the evaluation data and the exercise behavior data;
generating the patient eigenvector from the basic data, the initial evaluation data, and the medical record data of the patient, and the prescription eigenvector from the corresponding exercise prescription;
calculating a similarity between the patient eigenvector and the prescription eigenvector, determining a mean-square error of the similarity and the exercise effect, and determining the prescription generation model using the machine learning method with a target of minimizing the mean-square error; and
generating the exercise prescription using the prescription generation model.

Optionally, the personalized exercise guidance method may further include the following step after obtaining the basic data, the evaluation data, and the exercise behavior data of the patient, and generating the exercise prescription according to the basic data and the medical record data of the patient:
storing the basic data, the evaluation data, the exercise behavior data, the medical record data, the exercise prescription, and the exercise effect of the patient.

Optionally, the personalized exercise guidance method may further include the following step before generating the patient eigenvector from the basic data, the initial evaluation data, and the medical record data of the patient, and the prescription eigenvector from the corresponding exercise prescription:
performing data pre-processing on the basic data, the evaluation data, the exercise behavior data, the medical record data, and the exercise prescription. The data pre-processing may include: data cleaning and data normalization.

Optionally, the personalized exercise guidance method may further include the following step after generating the exercise prescription using the prescription generation model:
sending the generated exercise prescription to the doctor client;
sending, by the doctor client, the exercise prescription after confirmation to the server; and
sending, by the server, the exercise prescription after confirmation to the patient client.

According to the specific embodiments provided by the present disclosure, the present disclosure has the following technical effects:
According to the personalized exercise guidance system and method based on machine learning provided by the present disclosure, the patient eigenvector is generated from the basic data, the initial evaluation data, and the medical record data of the patient, and the prescription eigenvector is generated from the corresponding exercise prescription. Then, according to the patient eigenvector, the prescription eigenvector, and the exercise effect, the prescription generation model is constructed using the machine learning method, and according to the patient's own situation, the personalized exercise prescription is determined, that is, the current optimal basic principles of the exercise prescription and exercise video guidance courses are matched for the patient. This saves manual analysis time and repetitive labor, and lowers the threshold for participation in exercise therapy and rehabilitation.

### BRIEF DESCRIPTION OF THE DRAWINGS

To describe the technical solutions in embodiments of the present disclosure or in the prior art more clearly, the accompanying drawings required in the embodiments will be briefly described below. Apparently, the accompanying drawings in the following description show merely some embodiments of the present disclosure, and other drawings can be derived from these accompanying drawings by those of ordinary skill in the art without creative efforts.
FIG. 1 is a schematic structural diagram of a personalized exercise guidance system based on machine learning provided by the present disclosure;
FIG. 2 is an overall flow diagram provided by the present disclosure; and
FIG. 3 is a flow diagram of a personalized exercise guidance method based on machine learning provided by the present disclosure.

### DETAILED DESCRIPTION OF THE EMBODIMENTS

The technical solutions in the embodiments of the present disclosure will be described below clearly and completely with reference to the accompanying drawings in the embodiments of the present disclosure. Apparently, the described embodiments are merely some rather than all of the embodiments of the present disclosure. All other embodiments obtained by a person of ordinary skill in the art based on the embodiments of the present disclosure without creative efforts shall fall within the protection scope of the present disclosure.

An objective of the present disclosure is to provide a personalized exercise guidance system and method based on machine learning, which can improve the accuracy and pertinence of exercise guidance.

To make the above-mentioned objective, features, and advantages of the present disclosure clearer and more comprehensible, the present disclosure will be further described in detail below in conjunction with the accompanying drawings and specific embodiments.

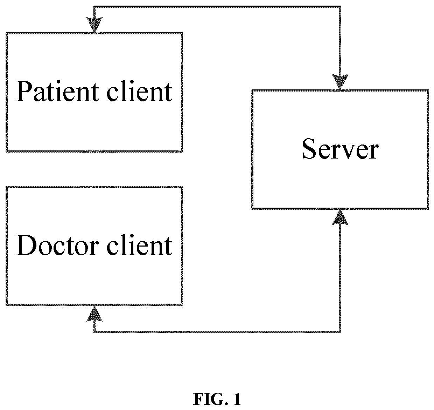

FIG. 1 is a schematic structural diagram of a personalized exercise guidance system based on machine learning provided by the present disclosure. As shown in FIG. 1, the personalized exercise guidance system based on machine learning includes: a server, a doctor client, and a patient client. The patient client is configured to obtain basic data, evaluation data, and exercise behavior data of a patient, and send the basic data, the evaluation data, and the exercise behavior data to the server. The basic data includes: gender, age, weight, living habits, exercise habits, diseases, pain conditions, and exercise risk information. The evaluation data includes: resting heart rate, cardiopulmonary endurance, upper limb and lower limb muscle strength, core muscle strength, upper limb and lower limb flexibility, balance, coordination, and musculoskeletal ability before and after an exercise prescription. The exercise behavior data includes: exercise time, exercise actions, heart rate during exercise, and physiological indicators before, during, and after exercise.

The doctor client is configured to determine the exercise prescription according to the basic data and medical record data of the patient, and upload the exercise prescription and the medical record data to the server. The medical record data includes: disease symptoms, physical signs, key indicators, and examination reports at different time points.

The server is configured to determine an exercise effect according the evaluation data and the exercise behavior data of the patient, generate a patient eigenvector from the basic data, initial evaluation data, and the medical record data of the patient, and a prescription eigenvector from the corresponding exercise prescription, and construct a prescription generation model using a machine learning method according to the patient eigenvector, the prescription eigenvector, and the exercise effect. The prescription generation model is configured to take the patient eigenvector as an input and the exercise prescription as an output. The exercise effect includes: 5: high efficiency, 4: marked effect, 3: effectiveness, 2: ineffectiveness, and 1: negative effect.

A process of constructing the prescription generation model using the machine learning method according to the patient eigenvector, the prescription eigenvector, and the exercise effect specifically includes the following steps.

A similarity between the patient eigenvector and the prescription eigenvector is calculated. A mean-square error of the similarity and the exercise effect is determined.

With a target of minimizing the mean-square error, mesh parameters of the model are learned, so as to obtain a calculation method from an original feature set to the eigenvector.

After the mesh parameters are obtained, the effect is verified through a verification set to monitor whether the model is over-fitted, and according to the verification results, the parameters are manually adjusted before training.

Through repeated training, and verification and adjustment of parameters, the mesh parameters with the optimal effect are obtained, and thus the optimal calculation method from the original feature set to the eigenvector is obtained.

After the optimal calculation method from the original feature set to the eigenvector is obtained, for the prescription customization of the new patient, the machine training results can be used to calculate the cosine similarity between the eigenvector of each prescription in a database and the eigenvector of the new patient. The prescription library is sorted according to the similarity, and the current optimal basic principles of the exercise prescription and exercise video guidance courses are matched for patient. After risk filtering, the prescription with the optimal effect is selected, and then prescribed to the patient for use.

In order to ensure the safety of the prescription of the patient, after the recommended prescription is generated through machine learning, the system performs safety risk verification on the prescription. These safety risk verification rules are derived from medical guidelines, which include exercise taboos and threshold ranges of various values. If a safety risk is found, a risk prompt message will be generated and sent to the doctor. After the doctor confirms and adjusts the prescription, it will be sent to the patient. If no safety risk is found, the prescription can be automatically sent to the patient or manually sent to the patient after confirmation by the doctor according to the settings of the doctor.

With the continuous increase of data, based on the continuous dynamic update of the accumulated database, machine learning is performed continuously, and the machine learning algorithm is used for analysis and the exercise prescription recommended by the system is continuously and dynamically optimized, so as to learn the exercise prescription with more excellent rehabilitation effect for different patients. The prescriptions recommended by the system will become more and more accurate, such that the exercise prescription recommendations by the system are infinitely close to the scheme with more excellent rehabilitation effect for individuals, the threshold for exercise rehabilitation is lowered, and more patients can benefit at low cost.

In order to ensure the consistency, effectiveness, and integrity of the data, the server includes: a data pre-processing module.

The data pre-processing module is configured to perform data pre-processing on the basic data, the evaluation data, the exercise behavior data, the medical record data, and the exercise prescription. The data pre-processing includes: data cleaning and data normalization.

A pre-processing process is as follows.

### 1. Removal of incomplete data

For the same patient, the basic patient information, patient evaluation data, exercise prescription data, exercise behavior data, and patient medical record data must be complete. For missing and incomplete data, the patient is marked with the corresponding data identification.

### 2. Removal of data containing outliers and invalid values

Using statistical methods, possible errors or outliers are identified and corresponding data identifications are established. For example, for the weight information of patients with certain characteristics, the fluctuation should be within a certain percentage threshold range before, during, and after the exercise prescription is executed, and the data exceeding the threshold range belongs to outliers. The setting of these threshold ranges should conform to medical logic and the content of published literature and medical guidelines.

### 3. Effect rating and data normalization

After data cleaning, the data is normalized. By comparing the behavior data of the patient during exercise and the medical record data of the patient before, during, and after exercise, and the execution effect of the exercise prescription is quantitatively analyzed, and the effect rating is given according to the requirements of the medical guidelines. The effect rating is planned to be divided into 5 levels, namely, 5: high efficiency, 4: marked effect, 3: effectiveness, 2: ineffectiveness, and 1: negative effect.

In order to reduce the computational complexity of machine learning, data normalization is performed on other data.

The server includes: a storage module.

The storage module is configured to store the basic data, the evaluation data, the exercise behavior data, the medical record data, the exercise prescription, and the exercise effect of the patient.

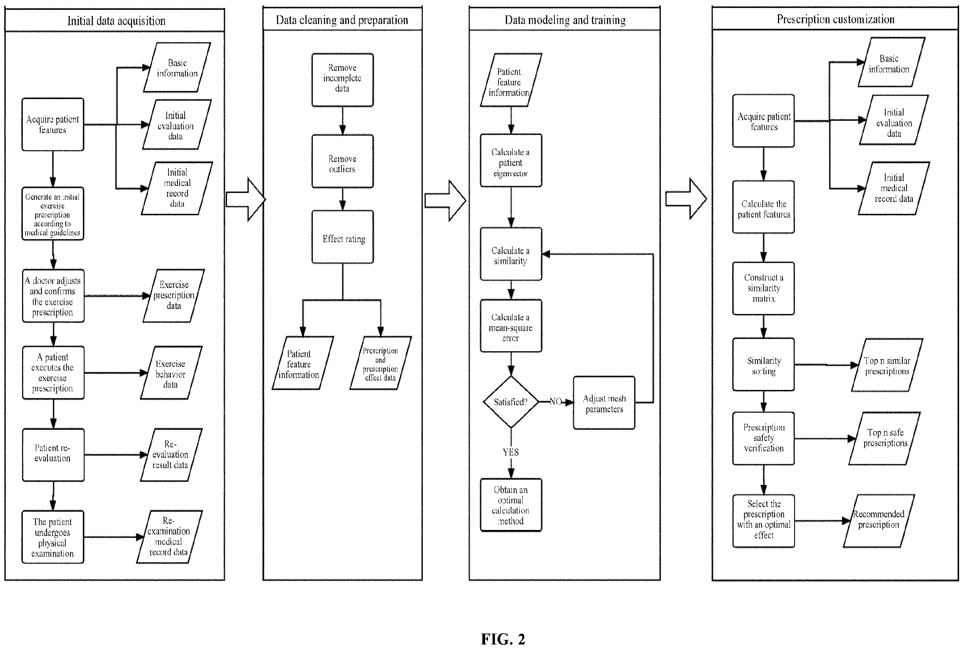

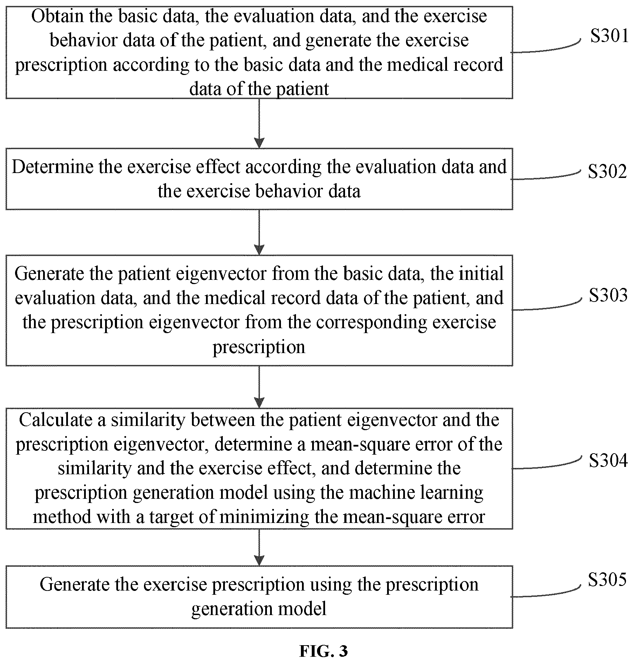

FIG. 2 is an overall flow diagram provided by the present disclosure. FIG. 3 is a flow diagram of a personalized exercise guidance method based on machine learning provided by the present disclosure. As shown in FIG. 2 and FIG. 3, the personalized exercise guidance method based on machine learning provided by the present disclosure, applied to the personalized exercise guidance system based on machine learning, includes the following steps.

S301, the basic data, the evaluation data, and the exercise behavior data of the patient is obtained, and the exercise prescription is generated according to the basic data and the medical record data of the patient.

After S301, the method further includes the following step.

The basic data, the evaluation data, the exercise behavior data, the medical record data, the exercise prescription, and the exercise effect of the patient are stored.

S302, the exercise effect is determined according the evaluation data and the exercise behavior data.

S303, the patient eigenvector is generated from the basic data, the initial evaluation data, and the medical record data of the patient, and the prescription eigenvector is generated from the corresponding exercise prescription.

Before S303, the method further includes the following step.

Data pre-processing is performed on the basic data, the evaluation data, the exercise behavior data, the medical record data, and the exercise prescription. The data pre-processing includes: data cleaning and data normalization.

S304, a similarity of the patient eigenvector and the prescription eigenvector is calculated, a mean-square error of the similarity and the exercise effect is determined, and the prescription generation model is determined using the machine learning method with a target of minimizing the mean-square error.

S305, the exercise prescription is generated using the prescription generation model.

After S305, the method further includes the following steps.

The generated exercise prescription is sent to the doctor client.

The doctor client sends the exercise prescription after confirmation to the server.

The server sends the exercise prescription after confirmation to the patient client.

The doctor client and the patient client are not only exercise rehabilitation management and execution platforms, but also patient data acquisition tools. The server is not only a data storage environment for machine learning, but also a data processing platform.

The patient evaluation data is acquired through the patient client and uploaded to the server and synchronized to the doctor client. The doctor formulates an exercise plan through the doctor client and sends it to the server. The information is synchronized to the patient client through the server and constitutes a playable exercise rehabilitation guidance course. The real-time feedback and regular re-evaluation results of the exercise rehabilitation of the patient are acquired through the patient client and sent to the server. The server analyzes the evaluation-plan-effect correlation based on all the acquired information, and finally establishes a system to automatically push the exercise prescription with the optimal rehabilitation effect according to the evaluation results.

To carry out exercise therapy and exercise rehabilitation, doctors need to have exercise science background and clinical medical background, and have corresponding clinical experience. The effect of the exercise prescription is closely related to the experience of the doctor. The intelligent exercise prescription generated by the system through machine learning integrates the experience of the doctor, and combines the clinically verified treatment and rehabilitation effects, which can provide clinical exercise therapy and rehabilitation guidance suggestions, help doctors optimize exercise therapy and rehabilitation schemes, and lower the threshold for exercise therapy and rehabilitation.

Each embodiment of the present specification is described in a progressive manner, each embodiment focuses on the difference from other embodiments, and the same and similar parts between the embodiments may refer to each other. Since the system disclosed in an embodiment corresponds to the method disclosed in another embodiment, the description is relatively simple, and reference can be made to the method description.

Specific examples are used herein to explain the principles and embodiments of the present disclosure. The foregoing description of the embodiments is merely intended to help understand the method of the present disclosure and its core ideas; besides, various modifications may be made by a person of ordinary skill in the art to specific embodiments and the scope of application in accordance with the ideas of the present disclosure. In conclusion, the content of the present description shall not be construed as limitations to the present disclosure.

## Claims

1. A personalized exercise guidance system based on machine learning, comprising: a server, a doctor client, and a patient client, wherein
the patient client is configured to obtain basic data, evaluation data, and exercise behavior data of a patient, and send the basic data, the evaluation data, and the exercise behavior data to the server; the basic data comprises: gender, age, weight, living habits, exercise habits, diseases, pain conditions, and exercise risk information; the evaluation data comprises: resting heart rate, cardiopulmonary endurance, upper limb and lower limb muscle strength, core muscle strength, upper limb and lower limb flexibility, balance, coordination, and musculoskeletal ability before and after an exercise prescription; and the exercise behavior data comprises: exercise time, exercise actions, heart rate during exercise, and physiological indicators before, during, and after exercise;
the doctor client is configured to determine the exercise prescription according to the basic data and medical record data of the patient, and upload the exercise prescription and the medical record data to the server; and the medical record data comprises: disease symptoms, physical signs, key indicators, and examination reports at different time points; and
the server is configured to determine an exercise effect according the evaluation data and the exercise behavior data of the patient, generate a patient eigenvector from the basic data, initial evaluation data, and the medical record data of the patient, and a prescription eigenvector from the corresponding exercise prescription, and construct a prescription generation model using a machine learning method according to the patient eigenvector, the prescription eigenvector, and the exercise effect; and the prescription generation model is configured to take the patient eigenvector as an input and the exercise prescription as an output.

2. The personalized exercise guidance system based on machine learning according to claim 1, wherein the server comprises: a data pre-processing module; and
the data pre-processing module is configured to perform data pre-processing on the basic data, the evaluation data, the exercise behavior data, the medical record data, and the exercise prescription; and the data pre-processing comprises: data cleaning and data normalization.

3. The personalized exercise guidance system based on machine learning according to claim 1, wherein the server comprises: a storage module; and
the storage module is configured to store the basic data, the evaluation data, the exercise behavior data, the medical record data, the exercise prescription, and the exercise effect of the patient.

4. A personalized exercise guidance method based on machine learning, applied to the personalized exercise guidance system based on machine learning according to any one of claims 1 to 3, comprising:
obtaining the basic data, the evaluation data, and the exercise behavior data of the patient, and generating the exercise prescription according to the basic data and the medical record data of the patient;
determining the exercise effect according the evaluation data and the exercise behavior data;
generating the patient eigenvector from the basic data, the initial evaluation data, and the medical record data of the patient, and the prescription eigenvector from the corresponding exercise prescription;
calculating a similarity between the patient eigenvector and the prescription eigenvector, determining a mean-square error of the similarity and the exercise effect, and determining the prescription generation model using the machine learning method with a target of minimizing the mean-square error; and
generating the exercise prescription using the prescription generation model.

5. The personalized exercise guidance method based on machine learning according to claim 4, further comprising the following step after obtaining the basic data, the evaluation data, and the exercise behavior data of the patient, and generating the exercise prescription according to the basic data and the medical record data of the patient:
storing the basic data, the evaluation data, the exercise behavior data, the medical record data, the exercise prescription, and the exercise effect of the patient.

6. The personalized exercise guidance method based on machine learning according to claim 4, further comprising the following step before generating the patient eigenvector from the basic data, the initial evaluation data, and the medical record data of the patient, and the prescription eigenvector from the corresponding exercise prescription:
performing data pre-processing on the basic data, the evaluation data, the exercise behavior data, the medical record data, and the exercise prescription, wherein the data pre-processing comprises: data cleaning and data normalization.

7. The personalized exercise guidance method based on machine learning according to claim 4, further comprising the following step after generating the exercise prescription using the prescription generation model:
sending the generated exercise prescription to the doctor client;
sending, by the doctor client, the exercise prescription after confirmation to the server; and
sending, by the server, the exercise prescription after confirmation to the patient client.
